# EUROPEAN PATENT APPLICATION

(11) **EP 2 937 346 A1**
(43) Date of publication of application: **28.10.2015**
(21) Application number: 14165887.2
(22) Date of filing: 24.04.2014
(51) Int. Cl.: C07D 405/04, A61K 31/505

(54) **Co-crystals of lapatinib**

(71) Applicant: F.I.S.- Fabbrica Italiana Sintetici S.p.A., 36075 Alte di Montecchio Maggiore (VI) (IT)
(72) Inventor: Tesson, Nicolas, 08902 L'Hospitalet de Llobregat (ES); Segade Rodriguez, Antonio, 08017 Barcelona (ES)
(74) Representative: Leganza, Alessandro

(57) **Abstract**

The present invention refers to co-crystals of the pharmaceutical active ingredient named Lapatinib and to processes for the preparation thereof and medical uses.

## Description

### Technical Field

Object of the present invention are co-crystals, processes for the preparation thereof and uses of the active pharmaceutical ingredient named Lapatinib.

### Background Art

Lapatinib is an active pharmaceutical ingredient used for the treatment of breast cancer and other solid tumors. It is used for the treatment of patients with advanced or metastatic breast cancer whose tumors overexpress HER2 and is currently on the market under the name Tykerb. According to the manufacturer's instructions, the commercial product Tykerb contains Lapatinib as the ditosylate monohydrate salt of formula (Ibis):

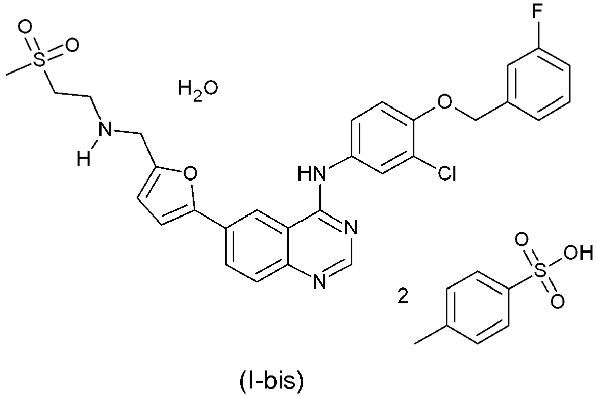

having the chemical name N-{3-chloro-4-[(3-fluorobenzyl)oxy]phenyl}-6-[5-({[2-(methylsulphonyl)ethyl]amino}methyl)furan-2-yl]quinazolin-4-amine bis(4-methylbenzenesulphonate) monohydrate, CAS RN 388082-78-8 and melting point 250-256°C.

The crystalline form of Lapatinib ditosylate monohydrate which, according to the statements of the author, appears to be that of the product currently on the market, is disclosed by Varlashkin P. on Power Diffr., Vol.24, No.3, September 2009, 250-253.

This substance can be prepared according to the teachings of the prior art such as, for example, those contained in EP1047694B1 or US7,157,466. In particular, in said last reference, examples 10 and 11 show the preparation of the ditosylate monohydrate salt starting from the anhydrous ditosylate salt.

Alternatively, Lapatinib and its ditosylate anhydrous and monohydrate form can be prepared according to the very efficient process described in the application EP13172771.1 and, moreover, the impurity orto-Lapatinib should be considered according to the teachings of EP2489661B.

In literature many solid forms of Lapatinib are disclosed, most of them are related to crystalline forms of Lapatinib ditosylate anhydrous (e.g. on IPCOM000199806 and IPCOM000204533) and few others are related to crystalline forms of Lapatinib base and Lapatinib ditosylate monohydrate. Moreover, many others solid forms of Lapatinib are disclosed being salts of Lapatinib (mainly disclosed on WO2010027848 and WO2008154469) and, furthermore, also are disclosed many solvates of Lapatinib ditosylate.

The application WO2010027848 has disclosed Lapatinib monotosylate, its crystalline form M1 and method for the preparation thereof.

Lapatinib ditosylate monohydrate is a non-hygroscopic yellow crystalline solid. The choice of this salt has been justified based on crystalline properties and good bioavailability. It was convincingly demonstrated that the particle size did not significantly impact dissolution. It is practically insoluble in aqueous media. (Tyverb : EPAR - Public assessment report)

Ratain and Cohen showed that the bioavailability of the new anti-cancer agent, Lapatinib, increased 325% when co-administered with a high fat meal when compared with fasting conditions. Lapatinib is a Class II drug (low solubility - good permeability) (Custodio J.M. at al., Adv Drug Deliv Rev., 2008 March 17; 60(6), 717-733 and M.J. Ratain, E.E. Cohen, The value meal: how to save $1,700 per month or more on lapatinib, J. Clin. Oncol. 25 (2007) 3397-3398).

Thus, a big drawback of Lapatinib and the known solid forms is the relatively low bioavailability, due to the very low solubility in water.

### Summary of invention

The problem addressed by the present invention regards the provision of an improved solid form of Lapatinib, which allows at least partially overcoming the aforementioned drawbacks with reference to the prior art, especially those related to the low bioavailability of Lapatinib.

This problem is resolved through a solid form of Lapatinib as outlined in the attached claims, whose definitions are an integral part of the present description.

Further characteristics and advantages of the solid forms according to the invention will be apparent from the description - indicated hereinafter - of preferred embodiments, provided by means of non-limiting example.

### Brief description of drawings

Figure 1 shows the Differential Scanning Calorimetry (DSC) curve of the co-crystal Lapatinib monotosylate : caproic acid (1:2) of formula (Form I);
Figure 2 shows the X-Ray Powder Diffraction (XPRD) diffratogram of the co-crystal Lapatinib monotosylate : caproic acid (1:2) of formula (Form I);
Figure 3 shows the Differential Scanning Calorimetry (DSC) curve of the co-crystal Lapatinib monotosylate : caprylic acid (1:2) of formula (Form II);
Figure 4 shows the X-Ray Powder Diffraction (XPRD) diffratogram of the co-crystal Lapatinib monotosylate : caprylic acid (1:2) of formula (Form II);
Figure 5 shows the Diff. Scan. Calorimetry (DSC) curve of the co-crystal Lapatinib monohydrochloride : adipic acid (1:1) of formula (Form III);
Figure 6 shows the X-Ray Pow. Dif. (XPRD) diffratogram of the co-crystal Lapatinib monohydrochloride : adipic acid (1:1) of formula (Form III);

### Description of embodiments

The present invention concerns co-crystal of Lapatinib selected in the group consisting of:
(a) Lapatinib monotosylate : caproic acid (1:2) of formula (Form I): 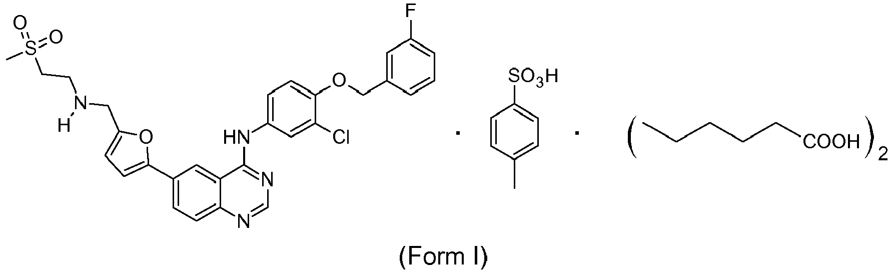
(b) Lapatinib monotosylate : caprylic acid (1:2) of formula (Form II): 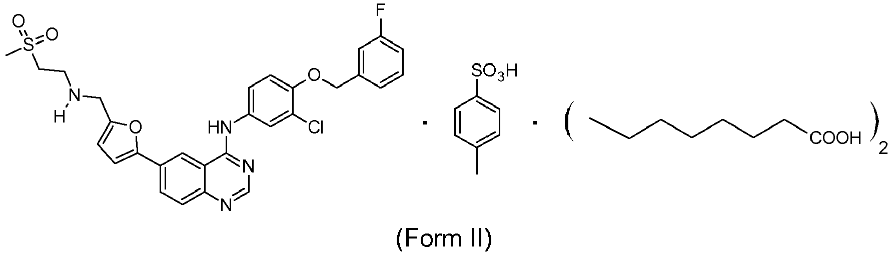
(c) Lapatinib monohydrochloride : adipic acid (1:1) of formula (Form III):

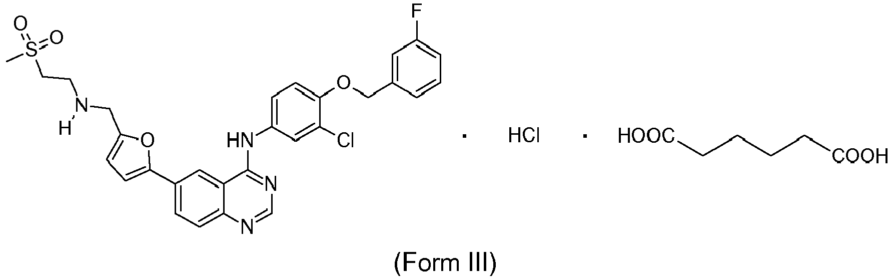

It has been indeed surprisingly found that is possible to prepare and isolate stable co-crystals of Lapatinib.

The large amount of publications directed to solid forms of Lapatinib testifies that a huge effort in terms of R&D has been carried out by many Companies to discover new solid forms of Lapatinib, nevertheless, co-crystals of Lapatinib or salt thereof, including co-crystals of Lapatinib ditosylate are not known up to now.

A co-crystal can be described as a crystalline structure formed by two different or more molecular entities where, in contrast to salts, the intermolecular interactions are not ionic, but weak forces like hydrogen bonding and π-π stacking.

In terms of general knowledge, the formation of co-crystals presents a highly unexpected and exceptional character, in contrast with the formation of different salts and also different crystalline forms (i.e. polymorphs) of a substance whose is nowadays possible at least to predict the existence by means of proper software. This concept is indeed confirmed by the following actual observation: are known co-crystals of only 30% of the APIs containing carboxylic groups marketed nowadays in the United States.

The three co-crystals of Lapatinib, object of the present invention, according to our knowledge, have never been disclosed or exemplified in prior art documents. More in general, until now, no co-crystal of Lapatinib are disclosed or exemplified in literature, i.e., the preparation of Lapatinib co-crystal is an exceptional and not expected event, especially if it is considered that Lapatinib is known since many years and many studies directed to the preparation processes and to new solid forms have been carried out in the past years providing a huge literature.

This is also confirmed by the fact that, although Lapatinib ditosylate is known to form numerous solvates, with solvents such as MeOH, IPA, NMP, dioxane, ACN, DMF, nitrobenzene and pyridine, all the experimental attempts to prepare co-crystals of Lapatinib ditosylated, using various coformers, solvents and crystallization techniques, failed.

The new discovered co-crystals of Lapatinib have the following composition:
(a) Lapatinib monotosylate : caproic acid (1:2),
(b) Lapatinib monotosylate : caprylic acid (1:2),
(c) Lapatinib monohydrochloride : adipic acid (1:1).

The special technical features that bounds these products is that they are co-crystals of Lapatinib.

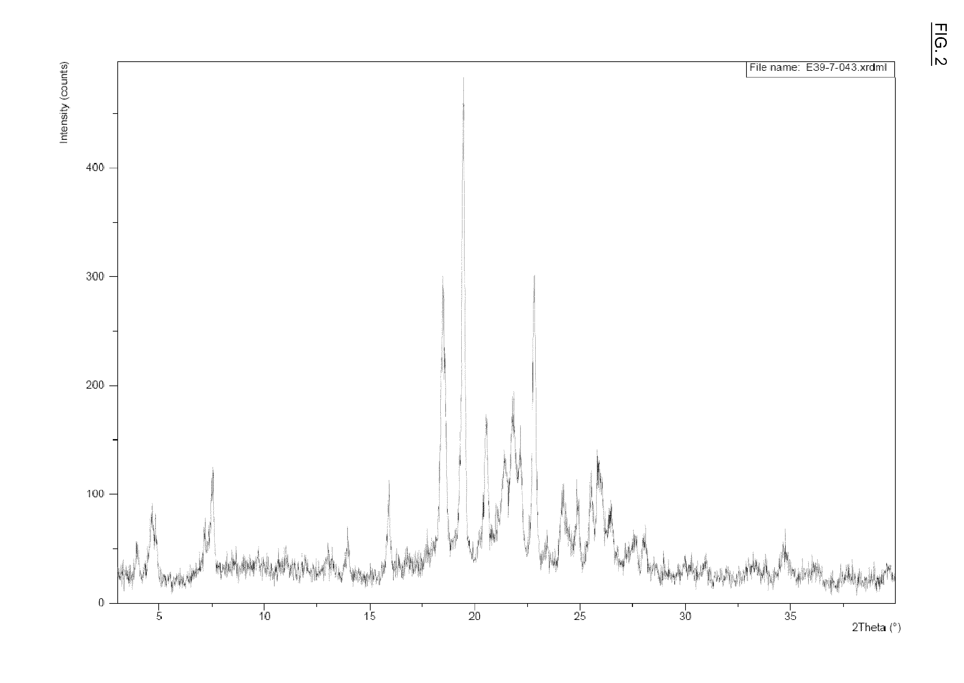

According to one embodiment, the co-crystal of Lapatinib being Lapatinib monotosylate : caproic acid (1:2) of formula (Form I) has a X-Ray Powder Diffractogram (XRPD) with characteristic peaks expressed in 2 Theta value of: 18,49, 19,47, 22,81 (+/-) 0,10 (See Fig. 2).

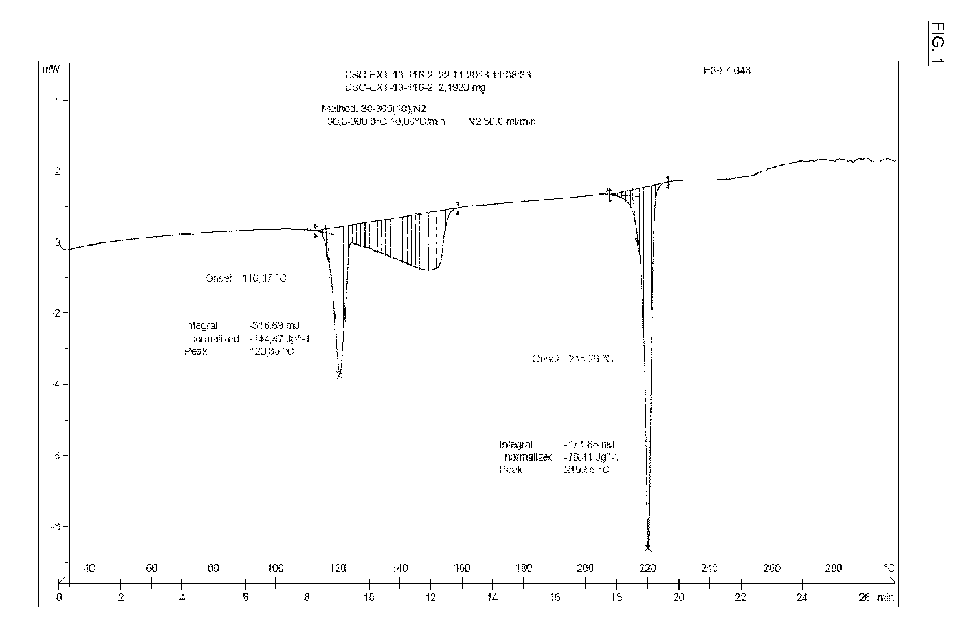

Co-crystal of Lapatinib being Lapatinib monotosylate : caproic acid (1:2) of formula (Form I) has an endothermic sharp peak with an onset at 116.17°C (See Fig. 1).

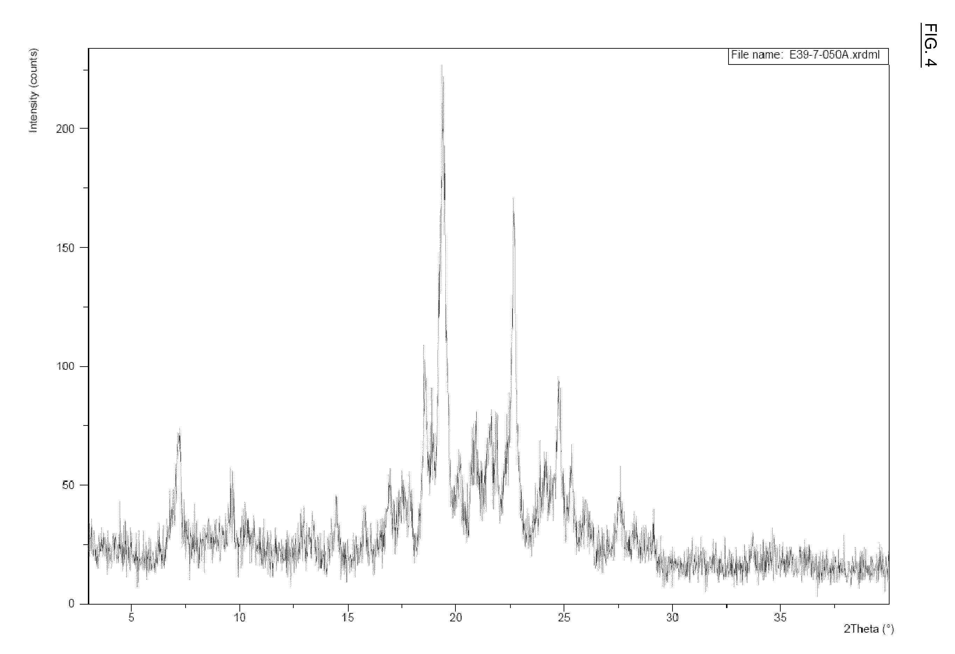

According to one embodiment, the co-crystal of Lapatinib being Lapatinib monotosylate : caprylic acid (1:2) of formula (Form II) has a X-Ray Powder Diffractogram (XRPD) with characteristic peaks expressed in 2 Theta value of: 18,55, 19,48, 22,71, 24,74 (+/-) 0,10 (See Fig. 4).

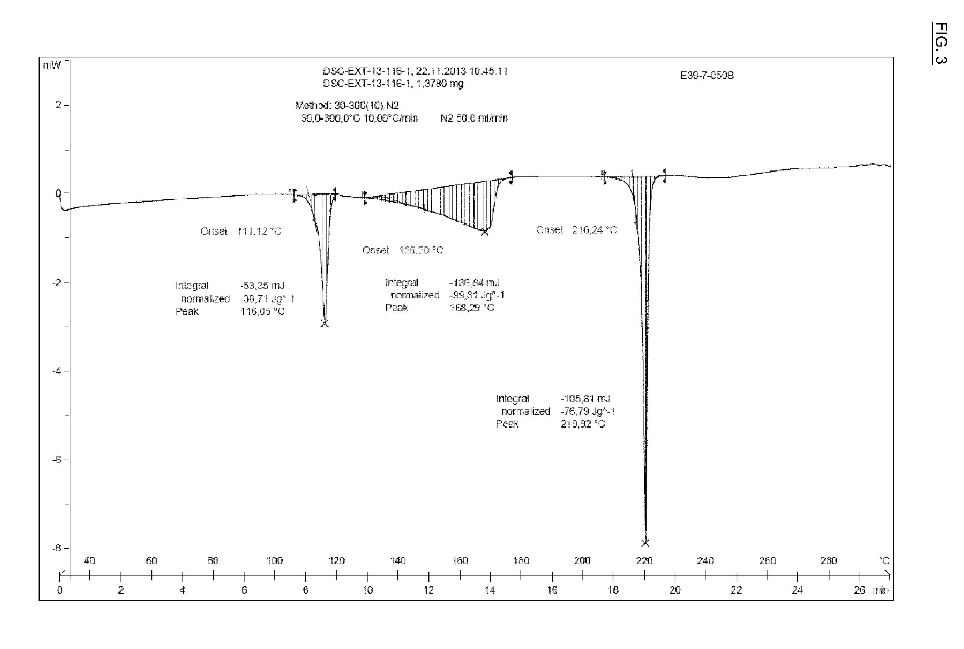

Co-crystal of Lapatinib being Lapatinib monotosylate : caprylic acid (1:2) of formula (Form II) has an endothermic sharp peak with an onset at 111.12°C (See Fig. 3).

According to one embodiment, the co-crystal of Lapatinib being Lapatinib monohydrochloride : adipic acid (1:1) of formula (Form III) has a X-Ray Powder Diffractogram (XRPD) with characteristic peaks expressed in 2 Theta value of: 15,77, 17,25, 19,00, 21,10, 21,70, 26,92 (+/-) 0,10.

Co-crystal of Lapatinib according to the claim 1 being Lapatinib monohydrochloride : adipic acid (1:1) of formula (Form III) has an endothermic sharp peak with an onset at 188.87°C.

The formation of a cocrystal, and not a salt, of formula (Form 1) and (Form 2) is confirmed by the stoichiometry: 2 basic points for three acids (1 p-toluenesulfonic acid and 2 aliphatic acids).

As further confirmation that the products of the present inventions are co-crystals and not simply salts, the criterion adopted by the FDA Guidelines of April 2013 (see below) is here reported: the active ingredient-excipient complex should be classified as co-crystal if ΔpKa of the two species is <1. Although in the literature it has not been possible to find the pKa of the second less basic function of Lapatinib, however, by comparison with other related molecules (Gefitinib and Erlotinib), it is possible to approximate a pKa 5.4. The pKa of caproic acid is 4.8, pKa of caprylic acid is 4.91 and pKa of adipic acid is 4.4, so that for all the products the FDA's criterion to classify them as co-crystals is satisfied.

Co-crystals do not involve structural modification of the parent molecules, therefore, in the case of designing co-crystals of marketed drugs, their development programs (including clinical trials) shall be significantly shorter and less risky than those of New Chemical Entities (NCEs).

As a result of the increasing relevance of the co-crystals as solid forms, in April 2013, the FDA issued a guidance for industry regarding "Regulatory Classification of Pharmaceutical Co-Crystals". This regulation paves the way to the use of co-crystals of APIs for new chemical entities and generic products. Indeed, since co-crystals are defined as a drug product intermediate (dissociable "API-excipient" molecular complexes), this consideration opens a new path for generic companies to file ANDA avoiding the 505 (b)(2) regulatory track. This could be applied also to the Lapatinib co-crystals of the present inventions, with evident advantages.

Moreover, according to P. H. Stahl and C. G. Wermuth, editors, Handbook of Pharmaceutical Salts: Properties, Selection and Use, Weinheim/Zürich:Wiley-VCH/VHCA, 2002, the conformers of the three co-crystals of the present invention are pharmaceutically acceptable acids, so that the co-crystals of the present invention have the advantage to be suitable for pharmaceutical compositions and medical treatments.

Furthermore, the fact that the bioavailability increases with a coadministration with high fat meal (see above) give a clear indication that the co-crystals of the present invention, with a monosalt of Lapatinib and a fatty acid as coformer, could lead to important advantageous properties in terms of highly increased bioavailability.

An other aspect of the present invention is the process for the preparation of the co-crystal of Lapatinib being
(a) Lapatinib monotosylate : caproic acid (1:2) of formula (Form I): 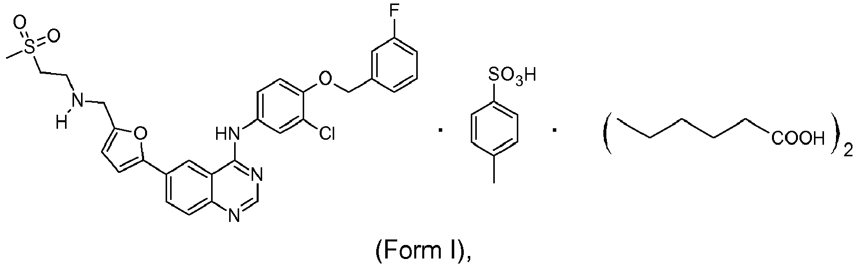 or being (b) Lapatinib monotosylate : caprylic acid (1:2) of formula (Form II): 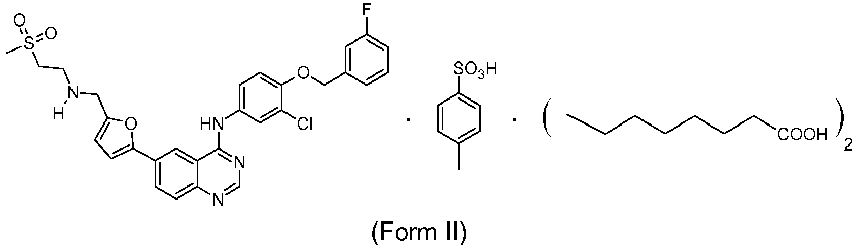 comprising the following steps:
   (A.) reaction of Lapatinib of formula (I) with paratoluensulfonic acid to provide Lapatinib monotosylate of formula (I - TsOH): 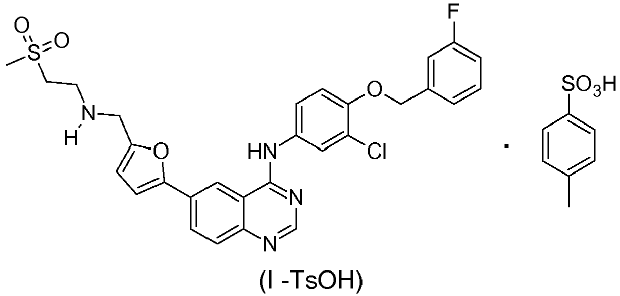
   (B.) treatment of Lapatinib monotosylate respectively with caproic acid or caprylic acid.

Lapatinib monotosylate salt was prepared from Lapatinib free base following the procedure reported in WO2010027848A2 (Example 1).

The preliminary solubility tests showed that Lapatinib monotosylate is highly insoluble in all the assayed solvents (H₂O, MeOH, EtOH, IPA, ACN, acetone, MIK, EtOAc, iBuOAc, TBME, THF, CH₂Cl₂, toluene, cyclohexane), even after heating to reflux at a 40 mL·g⁻¹ concentration. As Lapatinib monotosylate is a yellow colored product, relative solubility could be qualitatively assessed by the colour of the mother liquors, being MeOH the solvent which offered the higher solubilization.

Two new co-crystals of Lapatinib monotosylate salts were obtained with caproic and caprylic acids with a ratio 1:2 (confirmed by NMR). Therefore, the formation of a co-crystal (of a salt) is confirmed by the stoichiometry: 2 basic points for three acids (1 p-toluenesulfonic acid and 2 aliphatic acids).

The two co-crystals obtained with caproic acid and caprylic acids were both obtained by slurrying in water and were scaled up from milligrams to 15 grams of Lapatinib monotosylate with excellent yield.

The method of preparation of co-crystal of formula (Form I) and (Form II) is scalable on industrial plant since:
- it is a method in solution (slurrying),
- it employs ICH guideline solvent class III (water),
- it provides high yield (from 86% to almost quantitative).

Despite their preparation in water, the co-crystals of the invention do not incorporate water molecules in the crystalline structure as demonstrated by TGA.

According to a preferred embodiment of the process for the preparation of the co-crystal of Lapatinib monotosylate : caproic acid (1:2) of formula (Form I), the amount of caproic acid is between 2.5 and 3.0 equivalents, being preferred 2.7 equivalents, since it provides the complete transformation of Lapatinib monotosylate to co-crystal of formula (Form I). Indeed, with 2.1 equivalents of caproic acid the transformation was not complete after one night. The excess of caproic acid was successfully eliminated with the washing, as confirmed by ¹H-NMR.

According to a preferred embodiment of the process for the preparation of the co-crystal of Lapatinib monotosylate : caprylic acid (1:2) of formula (Form II) the solvent mixture is composed by water : EtOH 5:1. Indeed, using a molar ratio between Lapatinib monotosylate and caplylic acid of 1 : 2.2, said mixture afforded the desired co-crystal with a complete conversion. EtOH was chosen instead of MeOH because both gave similar results and EtOH is a class 3 ICH guideline solvent.

According to a preferred embodiment of the process for the preparation of the co-crystal of Lapatinib monotosylate : caprylic acid (1:2) of formula (Form II) the isolated product is washed three times with 2.5 volumes of mixture water:EtOH 5:1, since performing only two washings, a very little residual caprylic acid was detected. A third washing afforded Form II without residual caprylic acid (controlled by ¹H-NMR).

According to a preferred embodiment of the process for the preparation of the co-crystal of Lapatinib monotosylate : caprylic acid (1:2) of formula (Form II) the amount of caprylic acid employed is comprised in the range from 2.2 to 2.5 equivalents, preferably 2.3 equivalents. Indeed, at 4 g scale, 2.3 equivalents of caprylic acid were required for complete transformation of Lapatinib monotosylate to co-crystal of formula (Form II).

According to a an aspect of the process for the preparation of the co-crystal of Lapatinib monotosylate : caprylic acid (1:2) of formula (Form II) the experiment at 13.5 g scale had to be reprocessed because the solid obtained was not homogeneous (the diffraction of some samples showed complete transformation into Form II but others showed residual crystalline Lapatinib monotosulate). After reprocessing the solid with 1 extra equivalent of caprylic acid the solid had to be resuspended in 20 volumes of mixture water: EtOH, 5:1.

The preparation of the co-crystal having formula (Form II), at 13.5 g scale, includes two steps:
- formation of co-crystal of formula Form II with a small excess of Lapatinib monotosylate,
- reprocessing of the co-crystal to ensure complete conversion. (See examples 15 and 16).

In conclusion, increasing the scale of the experiments of the process for the preparation of the co-crystals of formula (Form I) and (Form II), some little modifications had to be made in order to achieve complete transformation of the starting products and to eliminate completely the excess of the coformers:
- in co-crystals of formula (Form I) and (Form II) a larger amount of coformer was required (2.3 or better 2.5 or best 2.7 equivalents instead of 2.1 equivalents).
- In Forms II additional washings (three instead of two) were needed in order to eliminate the excess of the coformer.

An other aspect of the present invention is the process for the preparation of the co-crystal of Lapatinib being Lapatinib monohydrochloride : adipic acid (1:1) of formula (Form III): 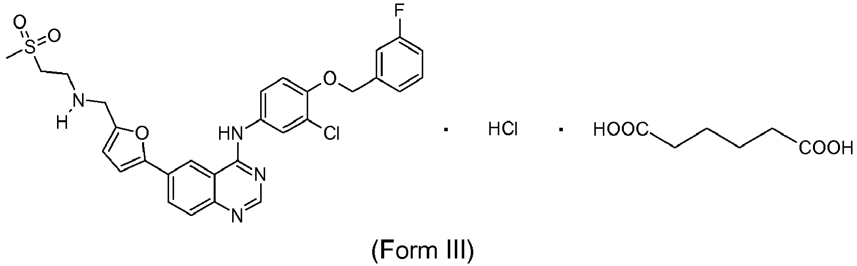 comprising the following steps:
(A.) reaction of Lapatinib of formula (I) with hydrochloric acid to provide Lapatinib monohydrochloride of formula (I - HCl): 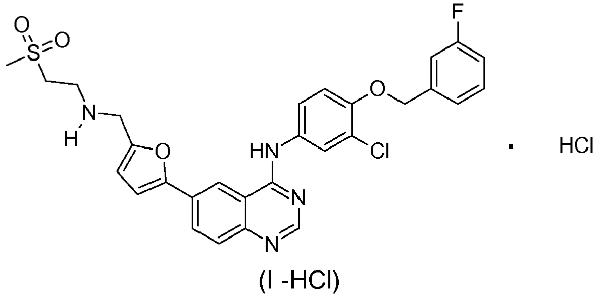
(B.) treatment of Lapatinib monohydrochloride with adipic acid.

According to a preferred embodiment of the process for the preparation of the co-crystal Lapatinib monohydrochloride : adipic acid (1:1) of formula (Form III), three washings (instead of one or two washings) are used to wash the solid product. Indeed, with three washings it is completely eliminated the excess of adipic acid (controlled by ¹H-NMR and XRPD).

Some slurryings in different alcoholic solvents were performed in order to check the stability of co-crystal of formula (Form III). It can be concluded that the stability of Form III is much higher in IPA (Isopropylalcohol) than in MeOH or EtOH. Therefore, IPA is preferred in order to perform the washing step of the co-crystal of formula (III).

The preparation of co-crystal of formula (Form III) was carried out at 5 g scale, in duplicate, to study the effect of the washings. Moreover, IPA washings were used instead of MeOH to decrease the risk of co-crystal dissociation. In this case, doing three washings of the product, both experiments furnished co-crystal of formula (Form III) as a pure form without excess of adipic acid (confirmed by XRPD and ¹H-NMR).

Thus, according to a preferred embodiment of the process for the preparation of the co-crystal Lapatinib monohydrochloride : adipic acid (1:1) of formula (Form III), in order to achieve complete transformation of the starting products and to eliminate the excess of the coformers:
- three washings of the isolated product were needed in order to eliminate the excess of the coformer,
- washings with IPA, instead that with MeOH, are beneficial for the preparation of the co-crystal of formula (Form III).

In conclusion, co-crystals of Lapatinib of formula (Form I) and (Form II) are prepared using Lapatinib monotosylate as starting product and co-crystal of Lapatinib of formula (Form III) is prepared starting from Lapatinib monohydrochloride. These three co-crystals were obtained by slurrying with very good yields at mg to multigrams scale.

The method of slurrying for the preparation of said co-crystals can be difficult to scale-up because the conversion rate depends highly on the stirring rate and stirring type, which can be very different at small and higher scale. Therefore, the different parameters of the slurrying (stirring, dilution, amount of coformers...) needed be optimized for each scale-up.

With the increase of the scale, a higher amount of coformers was necessary in order to obtain complete conversion (except with co-crystal of formula (Form III)). This problem of conversion could be due to the use of a mechanical stirring (no attrition effect as for magnetical stirring) or due to the formation of dense mixtures worsening the homogenization.

The use of higher amounts of coformer led to a problem of elimination of this excess and additional washings had to be carried out to obtain the pure crystal form. An additional suspension could to be performed in the case of Form II to eliminate the large excess of caprylic acid after the reprocessing step.

In the case of Form III, these additional washings caused the partial destruction of the co-crystal and another washing solvent had to be selected (IPA instead of MeOH) to avoid this dissociation.

Co-crystal of Lapatinib of formula (Form I) was prepared from Lapatinib monotosylate by slurrying in water in the presence of 2.7 eq. of caproic acid. 95% yield.

Co-crystal of Lapatinib of formula (Form II) was prepared from Lapatinib monotosylate by slurrying in water/EtOH (5:1) in the presence of 2.3 eq. of caprylic acid followed by a reprocessing in water/EtOH (5:1) in the presence of 1 eq. of caprylic acid. 86% overall yield.

Co-crystal of Lapatinib of formula (Form III) is prepared from Lapatinib monohydrochloride by slurrying in MeOH in the presence of 2 eq. of adipic acid followed by a washing with IPA. 87% yield.

According to the processes above described, the salts of Lapatinib with a monoprotic acid in stoichiometry (1:1) can be used for the preparation of co-crystals of Lapatinib. The monoprotic acid can be e.g. HCl, HBr, Benzensolfonic acid, p-Toluensofonic acid, formic acid, acetic acid, propionic acid, butirric acid, etc.

In particular, the salt Lapatinib monotosylate or Lapatinib monohydrochloride is useful for the preparation of co-crystals of Lapatinib.

More preferably, salts of Lapatinib with a monoprotic acid in stoichiometry (1:1), and in particular Lapatinib monotosylate or Lapatinib monohydrochloride, can be used for the preparation of co-crystals of Lapatinib and, in particular, for the preparation of the three co-crystals having formula (Form I), (Form II), (Form III).

The co-crystals of Lapatinib of the present invention having formula (Form I), (Form II), (Form III) are useful in medicine.

In particular, the co-crystal of Lapatinib of the present invention can be used in a method for treatment of breast cancer and other solid tumours.

More in particular, the co-crystal of Lapatinib of the present invention can be used for the treatment of patients with advanced or metastatic breast cancer whose tumors overexpress HER2 (ErbB2).

To employ the co-crystals of Lapatinib of the present invention for medical uses it is necessary to prepare pharmaceutical compositions comprising said a co-crystal of Lapatinib with one ore more excipients, carriers or diluents pharmaceutically acceptable.

The pharmaceutical compositions comprising the co-crystals of Lapatinib of the present invention can be orally administrated.

The pharmaceutical compositions comprising the co-crystals of Lapatinib of the present invention can be in the form of film-coated tablets and can comprise an amount of co-crystal equivalent to from 50 to 300 mg Lapatinib free base, preferably 250 mg.

The pharmaceutical compositions comprising the co-crystals of Lapatinib of the present invention can further comprise one or more of the following inactive ingredients: cellulose - microcrystalline, povidone (K30), croscarmellose sodium, sodium starch glycolate and magnesium stearate.

The stabilities studies of co-crystals of Lapatinib of the present invention shown that said co-crystals are stable.

### EXPERIMENTAL PART

### Example 1 - Preparation of Lapatinib monotosylate of formula (I, 1 TsOH).

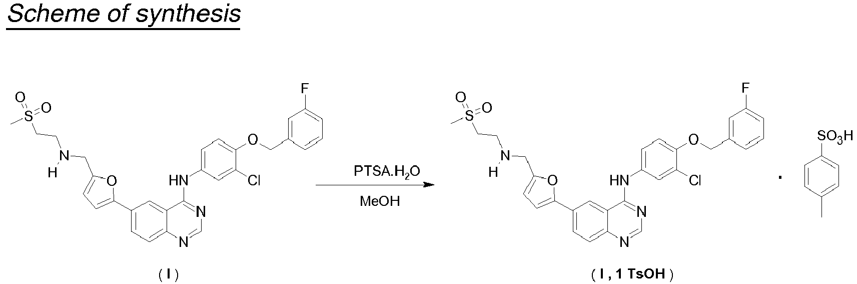

Lapatinib free base of formula (I) (2.00 g, 3.44 mmol) was suspended in MeOH (80 mL, 40 mL·g⁻¹). To the brown/yellow suspension, PTSA·H₂O (0.67 g, 3.52 mmol, 1.02 eq) was added and the resulting bright yellow mixture was stirred at r.t. overnight. The solid was filtered, washed with MeOH (5 mL, 2.5 mL·g⁻¹) and dried under vacuum at r.t., affording Lapatinib monotosylate of formula (I, 1 TsOH) (2.46 g, 3.27 mmol, 95% molar yield) as a yellow solid.

### Characterization:

¹H-NMR (*d₆*-DMSO, 400 MHz): δ (ppm) = 9.99 (s, 1H), 8.85 (s, 1H), 8.60 (s, 1 H), 8.23 (dd, J = 8.8, 1.6, 1 H), 7.99 (d, J = 2.4, 1 H), 7.86 (d, J = 8.8, 1 H), 7.73 (dd, J = 8.8, 2.4, 1 H), 7.52-7.44 (m, 3H), 7.37-7.26 (m, 3H), 7.23-7.14 (m, 1H), 7.10 (d, J = 7.6, 2H), 6.84 (d, J = 2.4, 1H), 5.27 (s, 2H), 4.40 (s, 2H), 3.61-3.49 (m, 2H), 3.47-3.39 (m, 2H), 3.19-3.10 (m, 3H), 2.28 (s, 3H).

XRPD: 2theta (θ) = 5.2, 6.0, 7.4, 8.6, 9.1, 10.0, 12.7, 14.8, 15.7, 17.6, 18.6, 19.8, 21.5, 22.2, 23.3, 23.9.

### Example 2 - Preparation of co-crystal Lapatinib monotosylate : caproic acid (1:2) of formula (Form I):

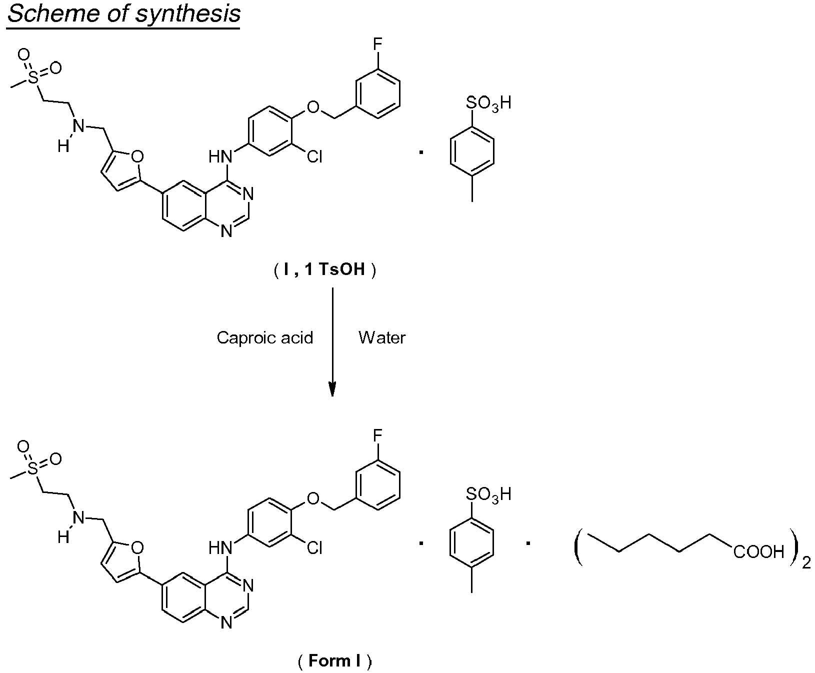

Lapatinib monotosylate of formula (I, 1 TsOH) as prepared in example 1 (0.20 g, 0.266 mmol) was suspended in H₂O (4 mL, 20 mL·g⁻¹). Caproic acid (0.07 mL, 0.559 mmol, 2.1 eq.) was added and the resulting suspension was stirred at r.t. overnight. The solid was filtered, washed with water and dried under vacuum at r.t., affording the desired cocrystal (0.27 g, quantitative yield) as an off-white solid.

Form I obtained by slurrying in water was characterized by several techniques:

- ¹H-NMR: Proton nuclear magnetic resonance analyses were recorded in deuterated dimethyl sulfoxide (DMSO-*d₆*) in a Varian Mercury 400 spectrometer, equipped with a broadband probe ATB 1 H/19F/X of 5 mm. Spectra were acquired dissolving 5-10 mg of sample in 0.6 mL of deuterated solvent.

¹H-NMR (*d₆*-DMSO, 400 MHz): δ (ppm) = 11.72 (bs, 1H), 9.98 (s, 1H), 9.31 (bs, 1 H), 8.84 (s, 1 H), 8.60 (s, 1 H), 8.22 (dd, J = 8.8, 1.6, 1 H), 7.98 (d, J = 2.4, 1 H), 7.86 (d, J = 8.8, 1 H), 7.72 (dd, J = 8.8, 2.4, 1 H), 7.50-7.44 (m, 3H), 7.34-7.27 (m, 3H), 7.20-7.14 (m, 2H), 7.09 (d, J = 8.0, 2H), 6.83 (d, J = 3.2, 1 H), 5.26 (s, 2H), 4.39 (s, 2H), 3.56-3.52 (m, 2H), 3.44-3.40 (m, 2H), 3.12 (s, 3H), 2.28 (s, 3H), 2.18 (t, J = 7.2, 4H), 1.55-1.44 (m, 4H), 1.33-1.19 (m, 8H), 0.86 (t, J = 6.8, 6H).

- DSC: DSC analysis was recorded with a Mettler DSC822e. A sample of 2.1920 mg was weighed into a 40 µL aluminium crucible with a pinhole lid and was heated, under nitrogen (50 mL/min), at 10°C/min from 30 to 300°C.

Form I is characterized by an endothermic sharp peak with an onset at 116.17 °C measured by DSC analysis (10 °C/min). Visual observation of the crystal did not show a melting: therefore it could be a solid-solid transition or a melting with an instantaneous recrystallization to the monotosylate salt.

Subsequently, a broad endothermic peak that could be due to caproic acid evaporation was observed (enthalpy fusion and evaporation -144.47 J/g). Another endothermic sharp peak corresponding to the melting of Lapatinib monotosylate salt with an onset at 215.29 °C (enthalpy fusion -78.41 J/g) was observed. (See Figure 1 - DSC).

- TGA: Thermogravimetric analysis was recorded in a thermogravimetric analyzer Mettler TGA/SDTA851^{e}. A sample of 7.4451 mg was weighed into a 70 µL alumina crucible with a pinhole lid and was heated at 10 °C/min from 30 to 300 °C, under nitrogen (50 mL/min). The TG analysis of Form I shows a 23.31% weight loss starting partially before the melting point (between 75 °C and 185 °C). This loss of weight could come from the evaporation of caproic acid (23.31% loss - calculated loss for 2 caproic acid molecules 23.57%).

- XRPD: XRPD analysis was performed using a PANalytical X'Pert diffractometer with Cu Kα radiation in Bragg-Brentano geometry. The system is equipped with a monodimensional, real time multiple strip detector. The diffractogram was recorded from 3° to 40° (2θ) at a scan rate of 17.6° per minute (see Figure 2). List of selected peaks (only peaks with relative intensity greater than or equal to 1% are indicated):

| Pos. [°2Th] | d-spacing [Å] | Rel. Int. [%] |
|---|---|---|
| 3,96 | 22,33 | 6 |
| 4,63 | 19,08 | 14 |
| 7,16 | 12,34 | 9 |
| 7,54 | 11,73 | 19 |
| 11,04 | 8,01 | 3 |
| 13,10 | 6,76 | 2 |
| 13,95 | 6,35 | 8 |
| 15,93 | 5,56 | 16 |
| 18,49 | 4,80 | 58 |
| 19,47 | 4,56 | 100 |
| 20,54 | 4,32 | 33 |
| 21,41 | 4,15 | 24 |
| 21,76 | 4,08 | 33 |
| 22,18 | 4,01 | 25 |
| 22,81 | 3,90 | 61 |
| 24,19 | 3,68 | 18 |
| 24,85 | 3,58 | 18 |
| 25,54 | 3,49 | 20 |
| 25,85 | 3,45 | 23 |
| 26,48 | 3,37 | 14 |
| 27,63 | 3,23 | 9 |
| 28,05 | 3,18 | 8 |
| 30,12 | 2,97 | 2 |
| 30,95 | 2,89 | 4 |
| 33,09 | 2,71 | 3 |
| 33,82 | 2,65 | 4 |
| 34,69 | 2,59 | 7 |
| 36,28 | 2,48 | 3 |
| 37,81 | 2,38 | 2 |

The co-crystal of Lapatinib being Lapatinib monotosylate : caproic acid (1:2) of formula (Form I) has a X-Ray Powder Diffractogram (XRPD) with characteristic peaks expressed in 2 Theta value of: 4,63 (m), 7,54 (m), 15,93 (m), 18,49 (s), 19,47 (vs), 20,54 (m), 21,41 (m), 21,76 (m), 22,18 (m), 22,81 (s), 24,19 (m), 24,85 (m), 25,54 (m), 25,85 (m), 26,48 (m), (+/-) 0,10; wherein (vs) = very strong intensity, (s) = strong intensity, (m) = medium intensity. In particular, the co-crystal having formula (Form I) has characteristic peaks expressed in 2 Theta value of: 18,49, 19,47, 22,81 (+/-) 0,10. (See Figure 2 - XRPD Diffractogram).

### Example 3 - Preparation of co-crystal Lapatinib monotosylate : caprylic acid (1:2) of formula (Form II):

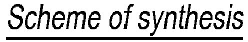

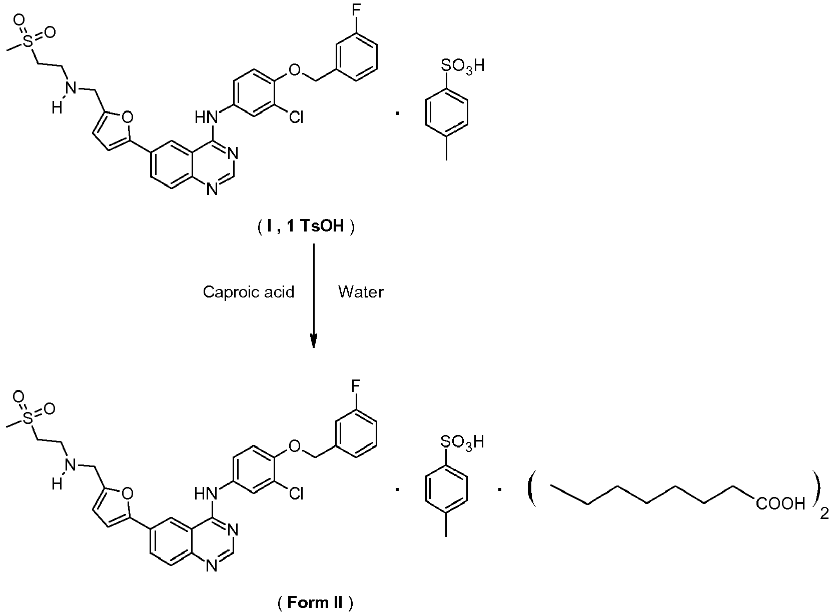

Lapatinib monotosylate (0.20 g, 0.266 mmol) was suspended in H₂O (4 mL, 20 mL·g⁻¹). Caprylic acid (83 mg, 0.576 mmol, 2.1 eq) was added and the resulting suspension was stirred at r.t. overnight. The solid was filtered, washed with water and dried under vacuum at r.t., affording the desired co-crystal (0.28 g, quantitative yield) as an off-white solid.

Similar results were obtained using a H₂O:MeOH 3:1 mixture as solvent.

Co-crystal of formula (Form II) obtained by slurrying in water was characterized by several techniques:

- ¹H-NMR: Proton nuclear magnetic resonance analyses were recorded in deuterated dimethyl sulfoxide (DMSO-*d₆*) in a Varian Mercury 400 spectrometer, equipped with a broadband probe ATB 1 H/19F/X of 5 mm. Spectra were acquired dissolving 5-10 mg of sample in 0.6 mL of deuterated solvent.

¹H-NMR (*d₆-*DMSO, 400 MHz): 5 (ppm) = 11.74 (bs, 1H), 9.98 (s, 1H), 8.85 (s, 1 H), 8.60 (s, 1 H), 8.23 (dd, J = 8.8, 1.6, 1 H), 7.99 (d, J = 2.4, 1 H), 7.87 (d, J = 8.8, 1 H), 7.73 (dd, J = 8.8, 2.4, 1 H), 7.53-7.43 (m, 3H), 7.37-7.26 (m, 3H), 7.23-7.14 (m, 2H), 7.10 (d, J = 8.0, 2H), 6.84 (d, J = 3.2, 1 H), 5.27 (s, 2H), 4.40 (s, 2H), 3.57-3.52 (m, 2H), 3.47-3.40 (m, 2H), 3.13 (s, 3H), 2.28 (s, 3H), 2.18 (t, J = 7.2, 4H), 1.52-1.43 (m, 4H), 1.33-1.19 (m, 12H), 0.85 (t, J = 6.8, 6H).

- DSC: DSC analysis was recorded with a Mettler DSC822e. A sample of 1.3780 mg was weighed into a 40 µL aluminium crucible with a pinhole lid and was heated, under nitrogen (50 mL/min), at 10 °C/min from 30 to 300 °C. A similar DSC profile to form I was observed. Form II is characterized by an endothermic sharp peak with an onset at 111.12°C (enthalpy fusion -38.71 J/g) measured by DSC analysis (10 °C/min) (see Figure 3). Visual observation of the crystal did not show a melting: therefore it could be a solid-solid transition or a melting with an instantaneous recrystallization to the monotosylate salt. Subsequently, a broad endothermic peak that could be due to caprylic acid evaporation was observed (enthalpy evaporation - 99.31 J/g). Another endothermic sharp peak corresponding to the melting of Lapatinib monotosylate salt with an onset at 216.24 °C (enthalpy fusion -76.79 J/g) was observed (see figure 3).

- TGA: Thermogravimetric analysis was recorded in a thermogravimetric analyzer Mettler TGA/SDTA851^{e}. A sample of 5.6640 mg was weighed into a 70 µL alumina crucible with a pinhole lid and was heated at 10°C/min from 30 to 300°C, under nitrogen (50 mL/min). The TG analysis of Form I shows a 27.37% loss weight starting partially before the melting point (between 91 °C and 195 °C). This loss of weight could come from the evaporation of caprylic acid (27.68% loss - calculated loss for 2 caproic acid molecules 27.68%).

- XRPD: XRPD analysis was performed using a PANalytical X'Pert diffractometer with Cu Kα radiation in Bragg-Brentano geometry. The system is equipped with a monodimensional, real time multiple strip detector. The diffractogram was recorded from 3° to 40° (2θ) at a scan rate of 17.6° per minute (see Figure 11). List of selected peaks (only peaks with relative intensity greater than or equal to 1% are indicated):

| Pos. [°2Th] | d-spacin g [Å] | Rel. Int. [%] |
|---|---|---|
| 7,25 | 12,20 | 30 |
| 9,74 | 9,08 | 13 |
| 13,16 | 6,73 | 4 |
| 14,49 | 6,11 | 12 |
| 15,78 | 5,62 | 10 |
| 16,91 | 5,24 | 18 |
| 17,76 | 4,99 | 13 |
| 18,55 | 4,78 | 41 |
| 19,48 | 4,56 | 100 |
| 20,16 | 4,41 | 25 |
| 20,93 | 4,24 | 32 |
| 21,58 | 4,12 | 33 |
| 21,88 | 4,06 | 31 |
| 22,71 | 3,92 | 89 |
| 24,06 | 3,70 | 21 |
| 24,74 | 3,60 | 46 |
| 25,34 | 3,52 | 25 |
| 26,08 | 3,42 | 12 |
| 27,52 | 3,24 | 18 |
| 28,95 | 3,08 | 6 |

The co-crystal of Lapatinib being Lapatinib monotosylate : caprylic acid (1:2) of formula (Form II) has a X-Ray Powder Diffractogram (XRPD) with characteristic peaks expressed in 2 Theta value of: 7,25 (m), 9,74 (m), 14,49 (m), 15,78 (m), 16,91 (m), 17,76 (m), 18,55 (s), 19,48 (vs), 20,16 (m), 20,93 (m), 21,58 (m), 21,88 (m), 22,71 (s), 24,06 (m), 24,74 (s), 25,34 (m), 26,08 (m), 27,52 (m), (+/-) 0,10; wherein (vs) = very strong intensity, (s) = strong intensity, (m) = medium intensity. In particular, the co-crystal having formula (Form II) has characteristic peaks expressed in 2 Theta value of: 18,55, 19,48, 22,71, 24,74 (+/-) 0,10. (See Figure 4 - XRPD Diffractogram).

XRPD comparison between forms I and II indicates that the crystalline forms are similar. However, this could be expected because the coformers are similar and the ratio between Lapatinib monotosylate salt and the coformers is identical (1:2).

### Example 4 - Preparation of Lapatinib monohydrochloride of formula (I, 1 HCl).

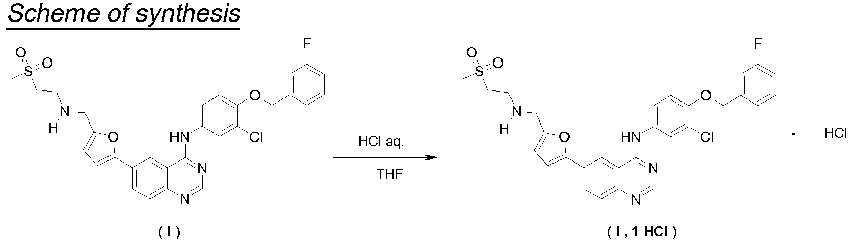

Lapatinib free base of formula (I) (1.00 g, 1.72 mmol)was suspended in THF (20 mL, 20 mL·g⁻¹). To the brown/yellow suspension, HCl 1 N (1.70 mL 1.70 mmol, 1.0 eq) was added and the resulting bright yellow mixture was stirred at r.t. overnight. The solid was filtered, washed with THF and dried under vacuum at r.t., affording Lapatinib monohydrochloride of formula (I, 1 HCl) (1.05 g, 1.70 mmol, 95% yield).

Characterization of Lapatinib monohydrochloride salt:

¹H-NMR: (*d₆*-DMSO, 400 MHz): δ (ppm) = 10.19 (s, 1H), 9.69 (bs, 1H), 9.20 (s, 1 H), 8.61 (s, 1 H), 8.23 (dd, J = 8.8, 1.6, 1 H), 8.14 (d, J = 2.4, 1 H), 7.89-7.83 (m, 2H), 7.50-7.44 (m, 1 H), 7.34-7.27 (m, 3H), 7.19-7.15 (m, 2H), 6.80 (d, J = 3.6, 1 H), 5.26 (s, 2H), 4.40 (s, 2H), 3.66-3.62 (m, 2H), 3.44-3.40 (m, 2H), 3.13 (m, 3H).

XRPD: Analysis of the monohydrochloride salt by XRPD indicates a form with a moderate crystallinity having peaks at 2theta values of 5,0, 7,5, 8,2, 10,0, 10,8, 11,7, 15,1, 15,5, 17,6, 20,3, 21,7.

### Example 5 - Preparation of Lapatinib dihydrochloride of formula (I, 2 HCl).

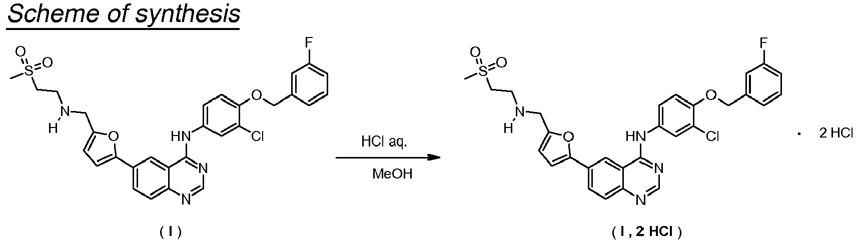

The Lapatinib dihydrochloride salt was prepared following the procedure reported in WO2010027848A2 (Example 6):

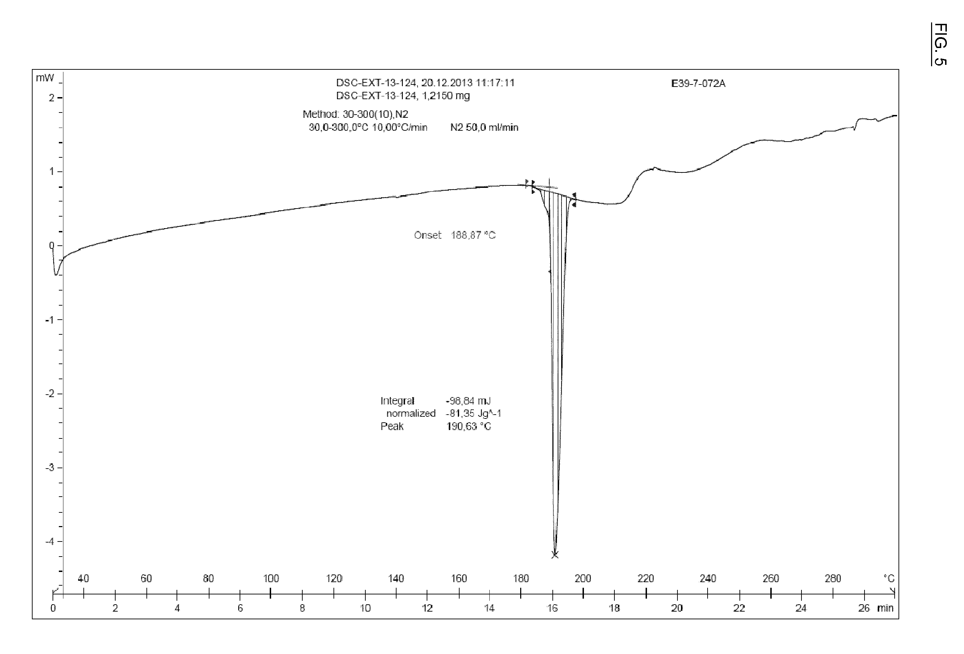

Lapatinib free base (1.01 g, 1.74 mmol) was suspended in MeOH (30 mL, 30 mL·g⁻¹). To the brown/yellow suspension, HCl 37% (0.33 mL, 3.9 mmol, 2.2 eq) was added and the resulting bright yellow mixture was stirred at r.t. overnight. The solid was filtered, and dried under vacuum at r.t., affording Lapatinib dihydrochloride of formula (I, 2 HCl) (1.06 g, 1.62 mmol, 93% yield). The diffractogram matched with the XRPD described in the application WO2010027848A2 for form C1 - Fig 5.1. and 5.2.

Comparison of ¹H-NMR and XRPD of the Lapatinib monohydrochloride salt prepared in example 4 with the Lapatinib dihydrochloride salt prepared in example 5 and with the Lapatinib free base confirms that Lapatinib monohydrochloride salt was effectively formed and it is not contaminated by the Lapatinib dihydrochloride or by the Lapatinib free base.

### Example 6 - Preparation of co-crystal Lapatinib monohydrochloride : adipic acid (1:1) of formula (Form III):

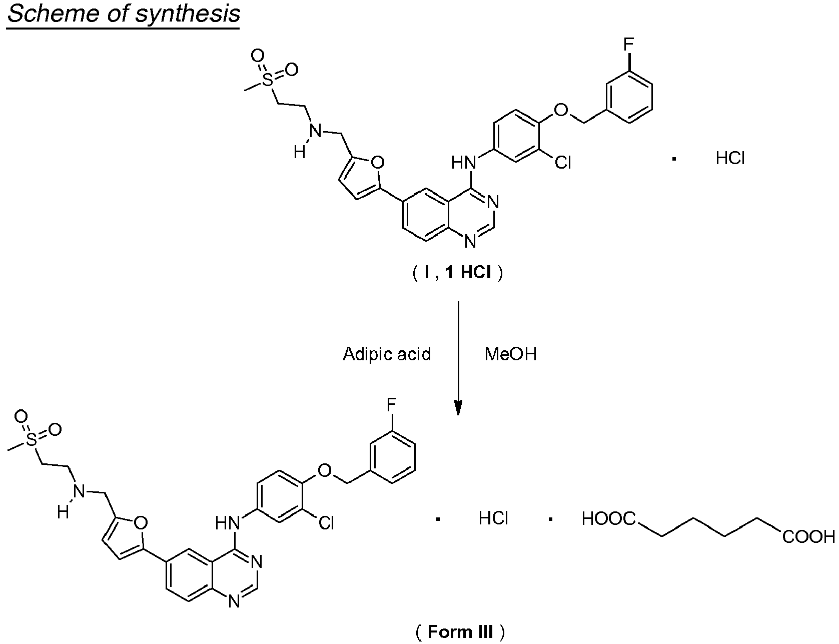

Lapatinib monohydrochloride (100 mg, 162 µmol) and adipic acid (50 mg, 342 µmol, 2.1 eq) were suspended in MeOH (2.0 mL). The resulting suspension (from yellow to off-white) was optionally seeded with previously obtained form III (A: seeded and C: without seeding) and the mixture was stirred overnight at r.t. The solid was filtered, washed with MeOH (0.4 mL) and dried under vacuum, affording in both cases the form III (105 mg, 85% yield) as a pale yellow solid.

Co-crystal of formula (Form III) obtained by slurrying in MeOH was characterized by several techniques:

- ¹H-NMR: Proton nuclear magnetic resonance analyses were recorded in deuterated dimethyl sulfoxide (DMSO-d6) in a Varian Mercury 400 spectrometer, equipped with a broadband probe ATB 1 H/19F/X of 5 mm. Spectra were acquired dissolving 5-10 mg of sample in 0.6 mL of deuterated solvent.

¹H-NMR (*d₆*-DMSO, 400 MHz): δ (ppm) = 10.15 (bs, 1H), 9.16 (s, 1H), 8.61 (s, 1 H), 8.23 (dd, J = 8.4, 1.6, 1 H), 8.13 (d, J = 2.3, 1 H), 7.93-7.81 (m, 2H), 7.52-7.43 (m, 1H), 7.37-7.26 (m, 3H), 7.22-7.15 (m, 2H), 6.81 (d, J = 3.5, 1 H), 5.27 (s, 2H), 4.40 (s, 2H), 3.70-3.59 (m, 2H), 3.42-3.38 (m, 2H), 3.13 (s, 3H), 2.20 (m, 4H), 1.49 (m, 4H).

The comparison of ¹H-NMR of cocrystal Form III and the Lapatinib·HCl salt indicates that there are not significant shifts in the different proton signals belonging to Lapatinib. Therefore, a salt of Lapatinib·HCl with adipic acid does not seem to be formed in DMSO.

- DSC: DSC analysis was recorded using a Mettler DSC822e instrument. A sample of 1.2150 mg was weighed into a 40 µL aluminum crucible with a pinhole lid and was heated, under nitrogen (50 mL/min), at 10°C/min from 30 to 300°C.

Form III is characterized by an endothermic sharp peak corresponding to the melting point with an onset at 188.87°C (fusion enthalpy -81.35 J/g), measured by DSC analysis (10 °C/min) (see Figure 5).

- TGA: Thermogravimetric analysis was recorded in a thermogravimetric analyzer Mettler TGA/SDTA851^{e}. A sample of 7.4451 mg was weighed into a 70 µL alumina crucible with a pinhole lid and was heated at 10 °C/min from 30 to 300°C, under nitrogen (50 mL/min).

The TG analysis of Form III only shows a negligible weight loss (0.16 %) before the melting point.

- XRPD: XRPD analysis was performed using a PANalytical X'Pert diffractometer with Cu Kα radiation in Bragg-Brentano geometry. The system is equipped with a monodimensional, real time multiple strip detector. The diffractogram was recorded from 3° to 40° (2θ) at a scan rate of 17.6° per minute (see Figure 7). Peaks with relative intensity greater than or equal to 1% are indicated in the Table below:

| Pos. [°2Th] | d-spacing [Å] | Rel. Int. [%] |
|---|---|---|
| 6,15 | 14,37 | 18 |
| 7,79 | 11,35 | 24 |
| 9,97 | 8,88 | 21 |
| 11,80 | 7,50 | 7 |
| 12,46 | 7,10 | 46 |
| 12,91 | 6,86 | 33 |
| 14,32 | 6,18 | 15 |
| 14,78 | 6,00 | 30 |
| 15,77 | 5,62 | 60 |
| 17,25 | 5,14 | 46 |
| 19,00 | 4,67 | 100 |
| 19,44 | 4,57 | 39 |
| 20,00 | 4,44 | 40 |
| 20,43 | 4,35 | 33 |
| 21,10 | 4,21 | 62 |
| 21,70 | 4,10 | 77 |
| 22,07 | 4,03 | 39 |
| 22,78 | 3,90 | 17 |
| 23,76 | 3,74 | 27 |
| 25,30 | 3,52 | 23 |
| 26,07 | 3,42 | 22 |
| 26,92 | 3,31 | 43 |
| 28,71 | 3,11 | 23 |
| 31,02 | 2,88 | 12 |
| 31,88 | 2,81 | 12 |

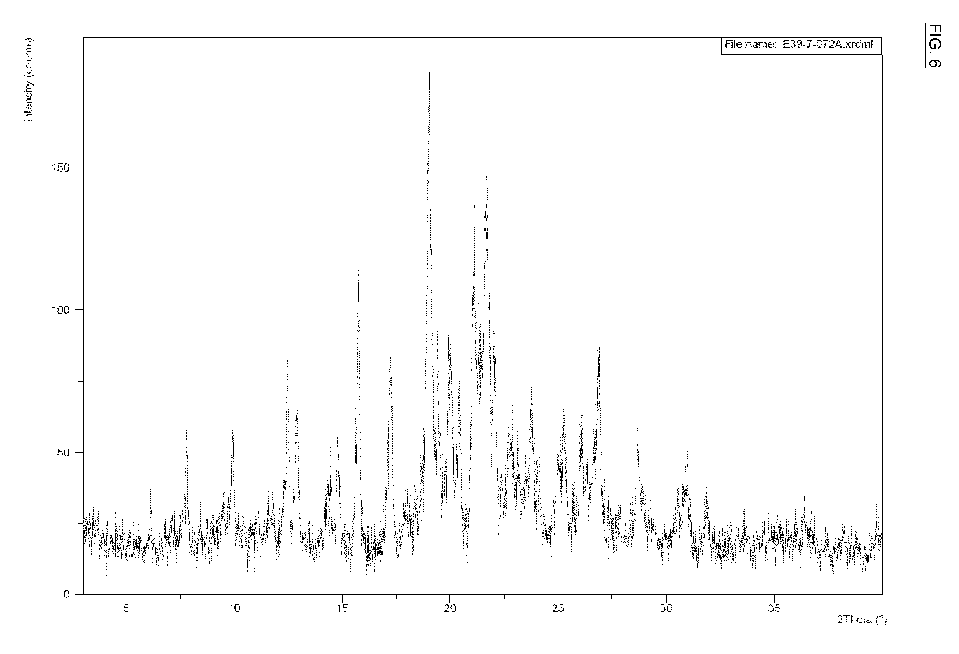

The co-crystal of Lapatinib being Lapatinib monohydrochloride : adipic acid (1:1) of formula (Form III) has a X-Ray Powder Diffractogram (XRPD) with characteristic peaks expressed in 2 Theta value of: 6,15 (m), 7,79 (m), 9,97 (m), 12,46 (s), 12,91 (m), 14,32 (m), 14,78 (m), 15,77 (s), 17,25 (s), 19,00 (vs), 19,44 (m), 20,00 (m), 20,43 (m), 21,10 (s), 21,70 (s), 22,07 (m), 22,78 (m), 23,76 (m), 25,30 (m), 26,07 (m), 26,92 (s), 28,71 (m), 31,02 (m), 31,88 (m), (+/-) 0,10; wherein (vs) = very strong intensity, (s) = strong intensity, (m) = medium intensity. In particular, the co-crystal having formula (Form III) has characteristic peaks expressed in 2 Theta value of: 15,77, 17,25, 19,00, 21,10, 21,70, 26,92 (+/-) 0,10. (See Figure 6 - XRPD Diffractogram).

### Example 7 - Preparation of Lapatinib monotosylate of formula (I, 1 TsOH).

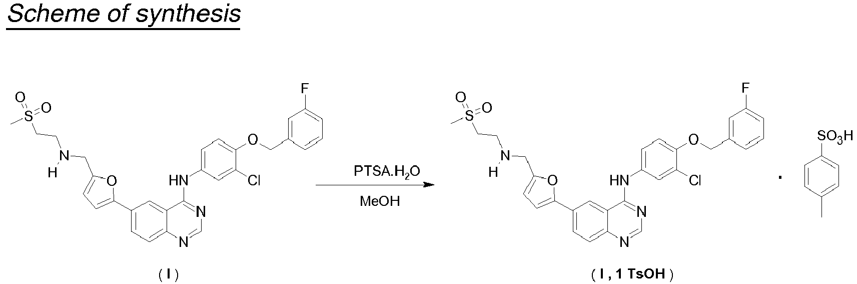

Lapatinib free base (8.00 g, 13.77 mmol) was suspended in MeOH (320 mL, 40 volumes). To the brown/yellow suspension, PTSA·H₂O (2.68 g, 14.09 mmol, 1.02 eq) was added and the resulting bright yellow mixture was stirred at r.t. overnight. The solid was filtered, washed with MeOH (40 mL, 5 volumes) and dried under vacuum at r.t., affording Lapatinib monotosylate (9.73 g, 12.92 mmol, 94% yield) as a yellow solid.

### Example 8 - Preparation of Lapatinib monohydrochloride of formula (I, 1 HCl).

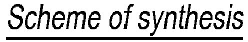

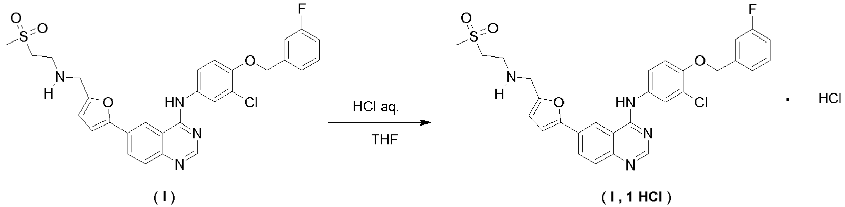

Lapatinib free base (13.00 g, 22.37 mmol) was suspended in THF (260 mL, 20 volumes). To the brown/yellow suspension, HCl 1 N (22.4 mL 22.40 mmol, 1.0 eq) was added and the resulting bright yellow mixture was stirred at r.t. overnight. The solid was filtered, washed with THF (26 mL, 2 volumes) and dried under vacuum at r.t., affording Lapatinib monohydrochloride (13.24 g, 21.44 mmol, 96% yield) as a yellow solid.

### Example 9 - Preparation of co-crystal Lapatinib monotosylate : caproic acid (1:2) of formula (Form I):

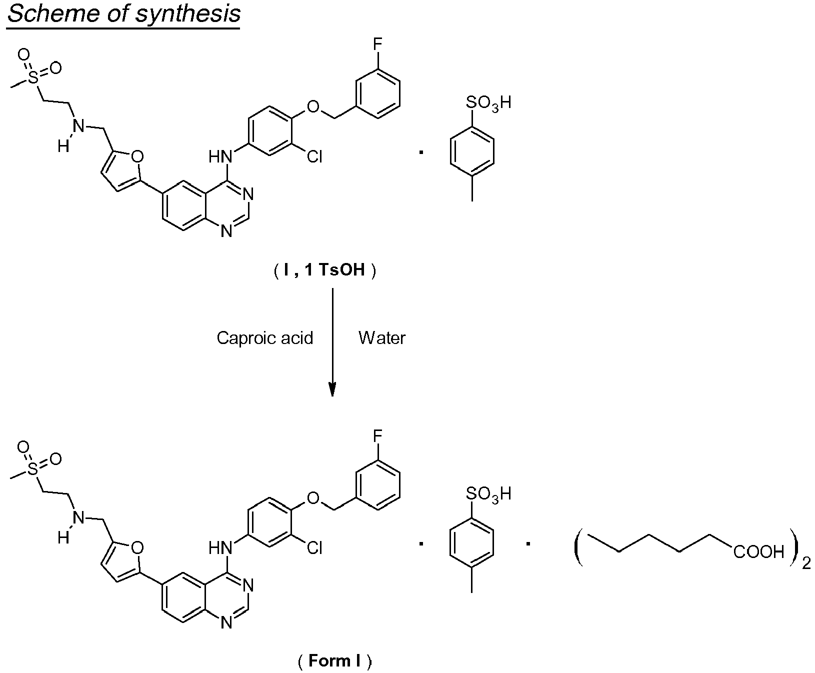

Lapatinib monotosylate (4.01 g, 5.32 mmol) was suspended in H₂O (80 mL, 20 volumes). Caproic acid (1.40 mL, 0.927 g/mL, 11.17 mmol, 2.1 eq) was added and the resulting suspension was stirred at r.t. overnight. A sample was filtered and analyzed by XRPD. Cocrystal Form I was obtained with residual Lapatinib monotosylate. Additional caproic acid (0.27 mL, 0.927 mg/mL, 2.15 mmol, 0.4 eq.) was added and the slurrying was stirred for a further 4 h. The solid was filtered, washed with water (10 mL, 2.5 volumes) and dried under vacuum at r.t., affording the desired cocrystal (4.84 g, 92% yield) as an off-white solid.

### Example 10 - Preparation of co-crystal Lapatinib monotosylate : caprylic acid (1:2) of formula (Form II):

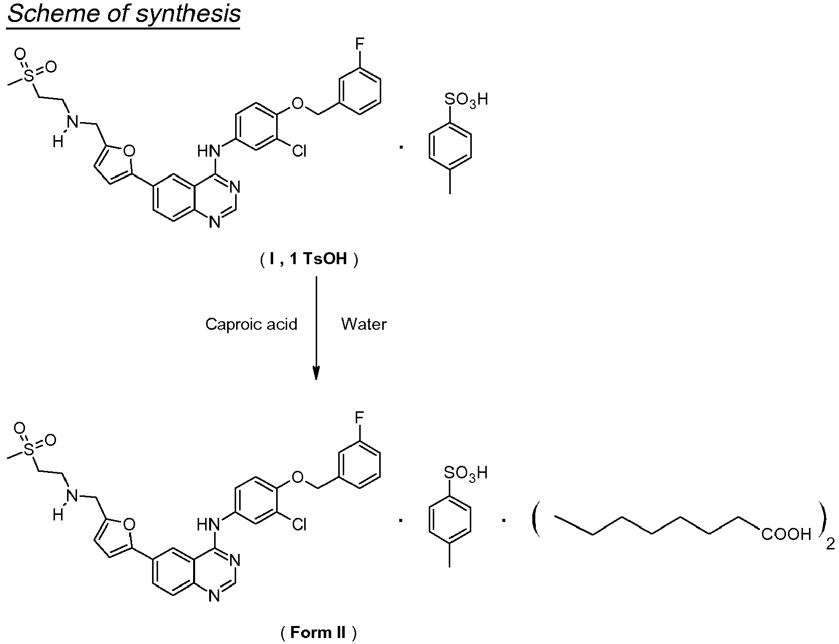

Lapatinib monotosylate (4.00 g, 5.31 mmol) and caprylic acid (1.85 mL, 0.910 g/mL, 11.67 mmol, 2.2 eq.) were suspended in H₂O:EtOH 5:1 (80 mL, 20 volumes). The resulting suspension was stirred at r.t. overnight. A sample was filtered and analyzed by XRPD. Cocrystal Form 2 was obtained with residual Lapatinib monotosylate. Additional caprylic acid (0.084 mL, 0.910 mg/mL, 0.53 mmol, 0.1 eq.) was added, and additional solvent (12 mL, 3 V, H₂O:EtOH 5:1), and the slurrying was stirred over the weekend. The solid was filtered, washed with water (3 x 12 mL, 3 x 3 volumes) and dried under vacuum at r.t., affording the desired cocrystal (5.08 g, 92% yield) as an off-white solid.

### Example 11 - Preparation of co-crystal Lapatinib monohydrochloride : adipic acid (1:1) of formula (Form III):

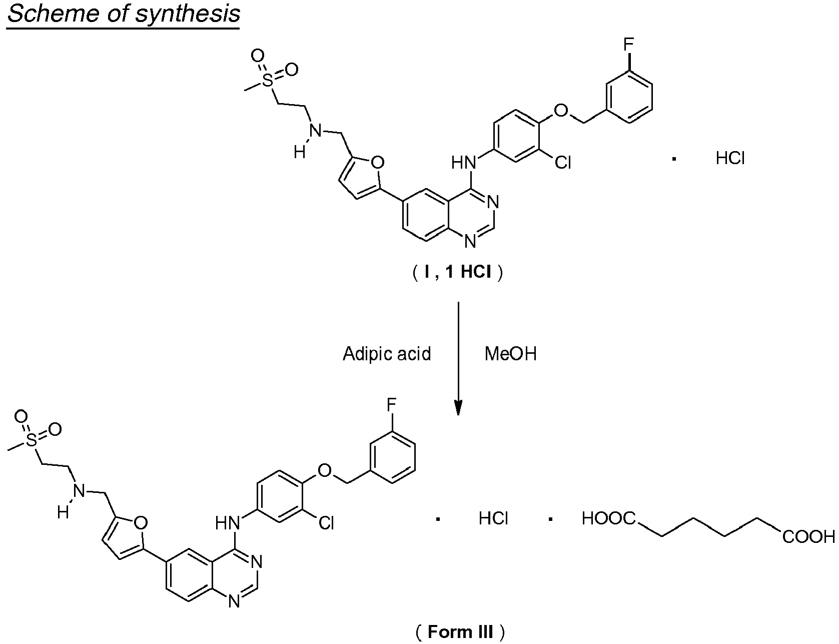

Lapatinib monohydrochloride (4.05 g, 6.56 mmol,) and adipic acid (2.02 g, 13.82 mmol, 2.1 eq) were suspended in MeOH (80 mL, 20 volumes). The resulting suspension was stirred overnight at r.t. The solid was filtered, washed with MeOH (3 x 12 mL, 3 x 3 volumes) and dried under vacuum, affording form III (4.26 g, 85% yield) as a pale yellow solid.

### Example 12 - Preparation of Lapatinib monotosylate of formula (I, 1 TsOH).

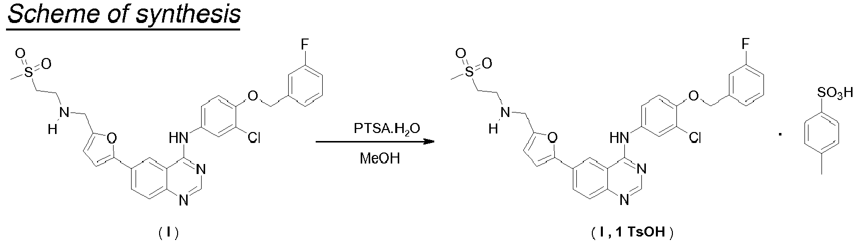

Lapatinib free base (17.26 g, 29.70 mmol) was suspended in MeOH (690 mL, 40 volumes). To the brown/yellow suspension, PTSA·H₂O (5.76 g, 30.28 mmol, 1.02 eq) was added and the resulting bright yellow mixture was stirred at RT overnight. The solid was filtered, washed with MeOH (86 mL, 5 volumes) and dried under vacuum at RT, affording Lapatinib monotosylate (21.02 g, 27.91 mmol, 94% yield) as a yellow solid.

### Example 13 - Preparation of Lapatinib monohydrochloride of formula (I, 1 HCl).

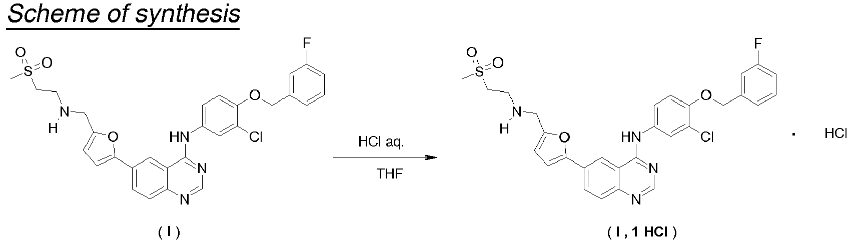

Lapatinib free base (13.00 g, 22.37 mmol) was suspended in THF (260 mL, 20 volumes). To the brown/yellow suspension, HCl 1 N (22.4 mL 22.40 mmol, 1.0 eq) was added and the resulting bright yellow mixture was stirred at RT overnight. The solid was filtered, washed with THF (26 mL, 2 volumes) and dried under vacuum at RT, affording Lapatinib monohydrochloride (13.24 g, 21.44 mmol, 96% yield) as a yellow solid.

### Example 14 - Preparation of co-crystal Lapatinib monotosylate : caproic acid (1:2) of formula (Form I):

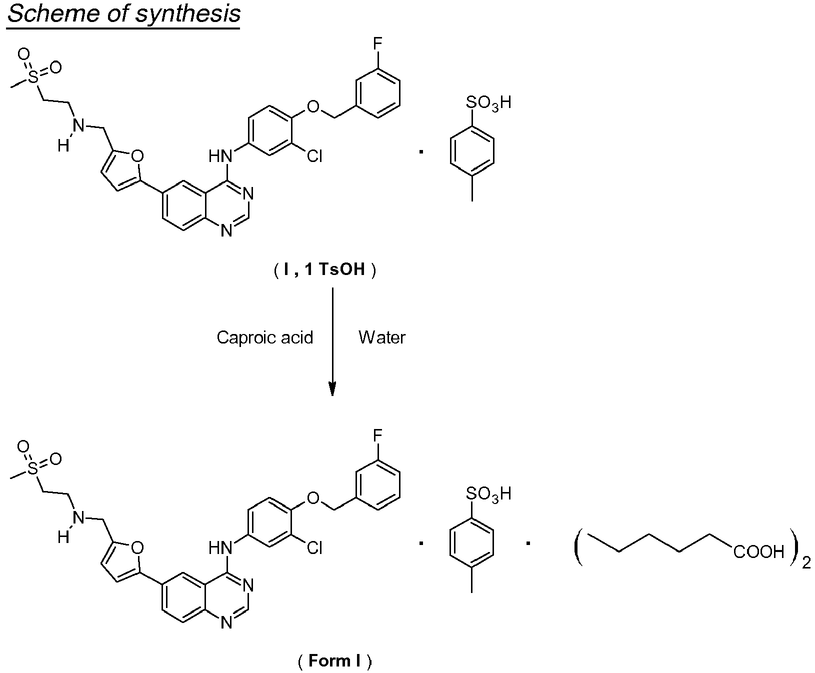

Lapatinib monotosylate (13.50 g, 17.92 mmol) was suspended in H₂O (270 mL, 20 volumes). Caproic acid (5.6 mL, 0.927 g/mL, 44.69 mmol, 2.5 eq) was added and the resulting suspension was stirred at RT overnight. A sample was filtered and analyzed by XRPD. Cocrystal Form I was obtained with residual Lapatinib monotosylate. Additional caproic acid (0.45 mL, 0.927 mg/mL, 3.59 mmol, 0.2 eq.) and more solvent (27 mL, 2 volumes) were added and the slurrying was stirred for 4 h (at this point the stirring was not good). The solid was filtered, washed with water (2 x 34 mL, 2 x 2.5 volumes) and dried under vacuum at RT, affording the desired cocrystal (16.83 g, 95% yield) as an off-white solid.

### Example 15 - Preparation of co-crystal Lapatinib monotosylate : caprylic acid (1:2) of formula (Form II):

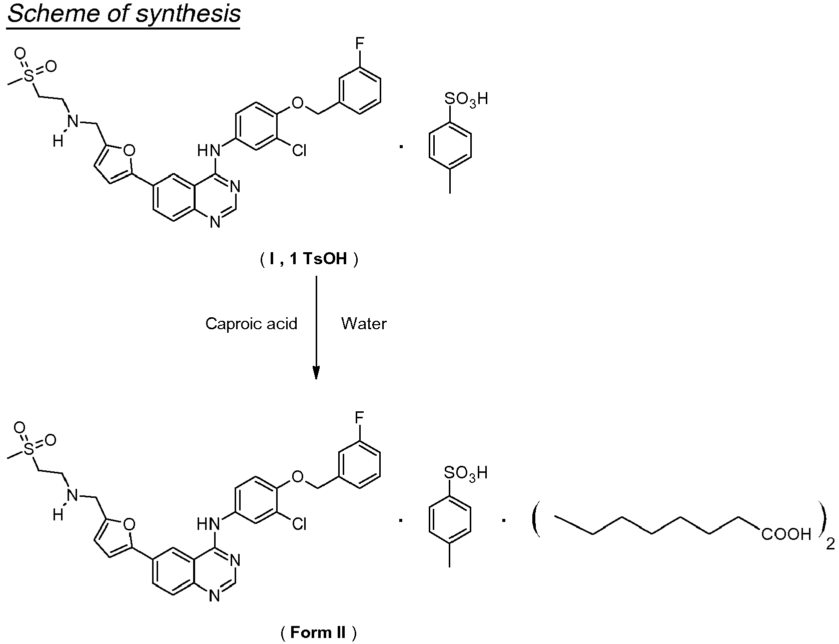

Lapatinib monotosylate (13.50 g, 17.92 mmol) and caprylic acid (6.5 mL, 0.910 g/mL, 41.02 mmol, 2.3 eq.) were suspended in H₂O:EtOH 5:1 (270 mL, 20 volumes). The resulting suspension was seeded with Form II and stirred at RT overnight. A sample was filtered and analyzed by XRPD. Co-crystal Form II was obtained with residual Lapatinib monotosylate. The slurrying was continued for 3.5 h (22 h in total) and complete transformation was checked by XRPD. The solid was filtered, washed with H₂O:EtOH 5:1 (3 x 40.5 mL, 3 x 3 volumes) and dried under vacuum at RT, affording the desired co-crystal (17.63 g, 94% yield) as an off-white solid.

Different samples of this solid were analyzed by XRPD and residual Lapatinib monotosylate was detected in some of them. As the solid was not homogeneous and residual starting product was detected the solid was reprocessed.

### Example 16 - Preparation of co-crystal Lapatinib monotosylate : caprylic acid (1:2) of formula (Form II) - reprocessing:

Lapatinib monotosylate:caprylic acid 1:2 (17.63, 16.92 mmol) and caprylic acid (2.7 mL, 0.910 g/mL, 17.04 mmol, 1 eq.) were suspended in H₂O:EtOH 5:1 (350 mL, 20 volumes). The resulting suspension was stirred at RT overnight. A sample was filtered and analyzed by XRPD confirming the transformation of residual Lapatinib monotosylate. The solid was filtered, washed with H₂O:EtOH 5:1 (8 x 70 mL, 8 x 4 volumes) and dried under vacuum at RT, affording the desired cocrystal as an off-white solid. The solid was analyzed by 1 H-NMR and an excess of almost 1 equivalent of caprylic acid was detected. The solid was resuspended twice in H₂O:EtOH 5:1 (350 mL, 20 volumes) until the excess of caprylic acid was completely removed from the solid. The desired cocrystal (16.09 g, 86% overall yield) was obtained as an off-white solid.

### Example 17 - Preparation of co-crystal Lapatinib monohydrochloride : adipic acid (1:1) of formula (Form III):

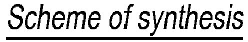

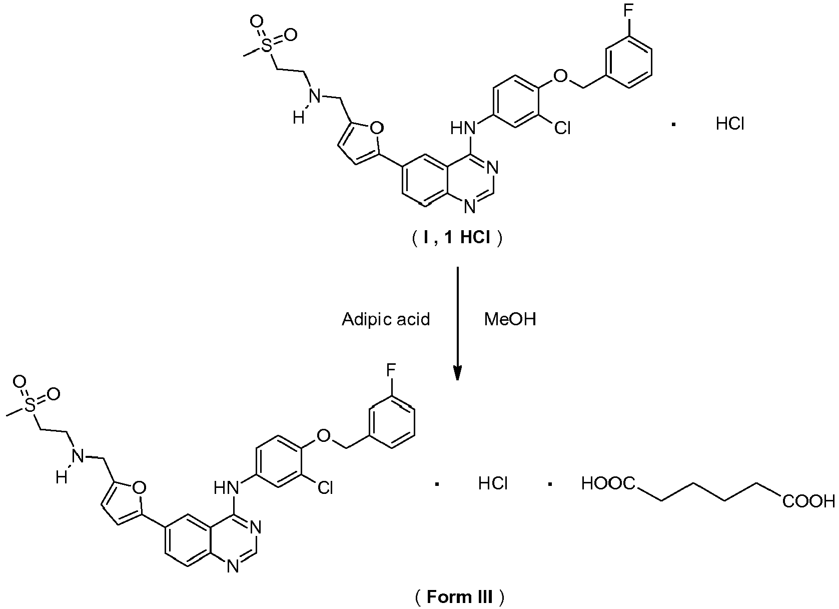

Lapatinib monohydrochloride (4.93 g, 7.98 mmol) and adipic acid (2.31 g, 15.81 mmol, 2 eq) were suspended in MeOH (98 mL, 20 volumes). The resulting suspension was stirred overnight at RT. The solid was filtered, washed with IPA (3 x 15 mL, 3 x 3 volumes) and dried under vacuum, affording Form III (5.33 g, 87% yield) as a pale yellow solid. This experiment furnished Form III as a pure form without excess of adipic acid (confirmed by XRPD and ¹H-NMR).

### Example 17 - Stability Study - Stability in water suspension

Stability of Form I, Form II and Form III in a water suspension was studied. The three suspensions were stirred at room temperature in 40 volumes of water and the transformation of the product was followed by XRPD at 1 hour, 4 hours and 24 hours. Form II is the most stable co-crystal, recovering pure Form II after 24 hours, while Form III and Form I are less stable in water; a little of Lapatinib Tosylate or Lapatinib hydrochloride was detected after 1 hour.

### Example 18 -Stability at controlled humidity and temperature conditions

Two different humidity/temperature conditions were selected for this study:
a. High humidity (94 RH%) at room temperature; and
b. Accelerated conditions of ICH guideline (40°C - 75 RH%).

### a. 94% RH at room temperature

No crystalline changes were detected by XRPD of any of the three forms after 41 days at 94% RH at room temperature. These co-crystals are quite stable at room temperature and high relative humidity.

### b. 74% RH at 40°C

No crystalline changes were detected by XRPD of any of the three forms after 11 days at 74% RH at 40°C.

After 23 days Lapatinib monotosylate is observed in the co-crystals of formula (Form II) (only traces) and co-crystals of formula (Form I) (a little amount).

The conclusions of the stability studies at controlled humidity and temperature conditions are: the co-crystals of Lapatinib of the present inventions are stable, thus suitable for pharmaceutical and medical uses. Furthermore, the ranking of stability is the following: co-crystal of formula (Form III) > co-crystal of formula (Form II) > co-crystal of formula (Form I), thus the co-crystal of formula (Form III) is the one more stable.

## Claims

1. Co-crystal of Lapatinib selected in the group consisting of:
(a) Lapatinib monotosylate : caproic acid (1:2) of formula (Form I): 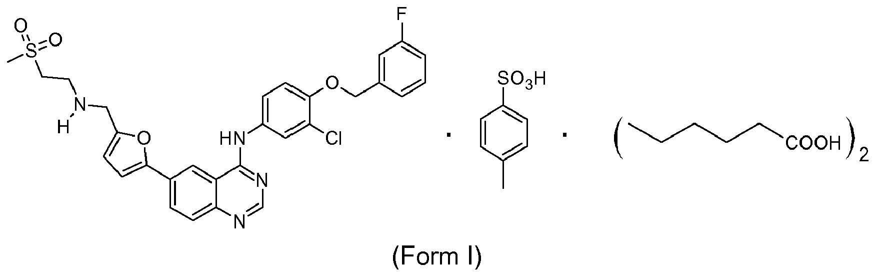
(b) Lapatinib monotosylate : caprylic acid (1:2) of formula (Form II): 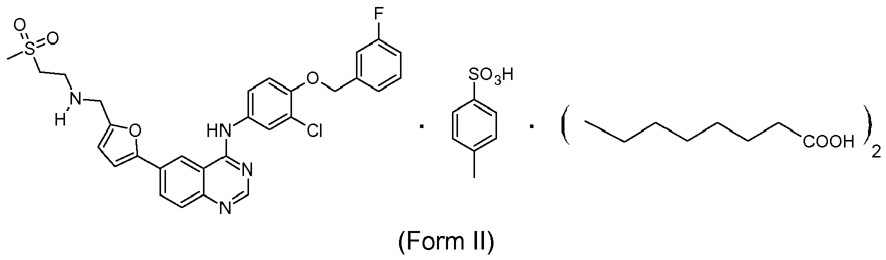
(c) Lapatinib monohydrochloride : adipic acid (1:1) of formula (Form III):

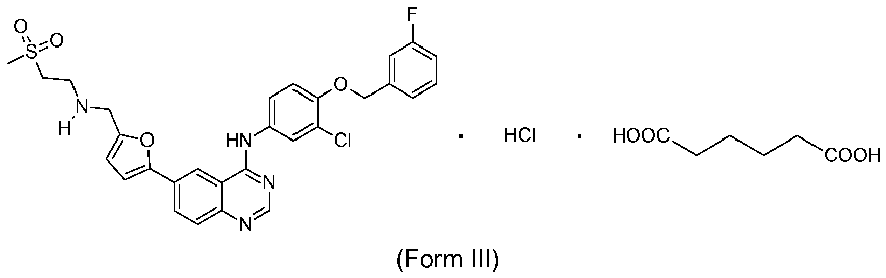

2. Co-crystal of Lapatinib according to the claim 1 being Lapatinib monotosylate : caproic acid (1:2) of formula (Form I) that has a X-Ray Powder Diffractogram (XRPD) with characteristic peaks expressed in 2 Theta value of: 18,49, 19,47, 22,81 (+/-) 0,10.

3. Co-crystal of Lapatinib according to the claim 1 being Lapatinib monotosylate : caproic acid (1:2) of formula (Form I) having an endothermic sharp peak with an onset at 116.17°C.

4. Co-crystal of Lapatinib according to the claim 1 being Lapatinib monotosylate : caprylic acid (1:2) of formula (Form II) that has a X-Ray Powder Diffractogram (XRPD) with characteristic peaks expressed in 2 Theta value of: 18,55, 19,48, 22,71, 24,74 (+/-) 0,10.

5. Co-crystal of Lapatinib according to the claim 1 being Lapatinib monotosylate : caprylic acid (1:2) of formula (Form II) having an endothermic sharp peak with an onset at 111.12°C.

6. Co-crystal of Lapatinib according to the claim 1 being Lapatinib monohydrochloride : adipic acid (1:1) of formula (Form III) that has a X-Ray Powder Diffractogram (XRPD) with characteristic peaks expressed in 2 Theta value of: 15,77, 17,25, 19,00, 21,10, 21,70, 26,92 (+/-) 0,10.

7. Co-crystal of Lapatinib according to the claim 1 being Lapatinib monohydrochloride : adipic acid (1:1) of formula (Form III) having an endothermic sharp peak with an onset at 188.87°C.

8. Process for the preparation of the co-crystal of Lapatinib being
(a) Lapatinib monotosylate : caproic acid (1:2) of formula (Form I): 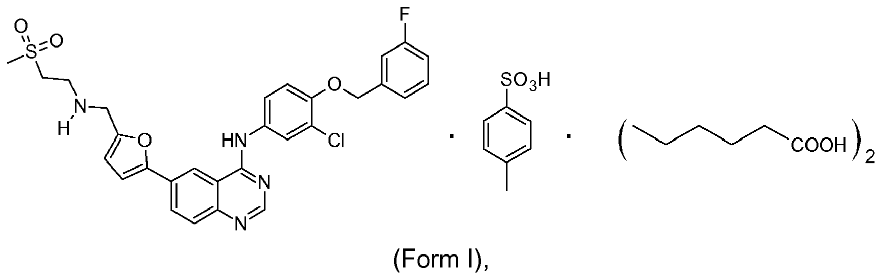 or being
(b) Lapatinib monotosylate : caprylic acid (1:2) of formula (Form II): 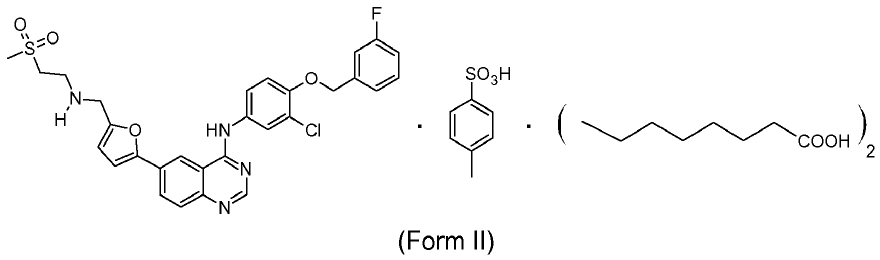 comprising the following steps:
(A.) reaction of Lapatinib of formula (I) with paratoluensulfonic acid to provide Lapatinib monotosylate of formula (I - TsOH): 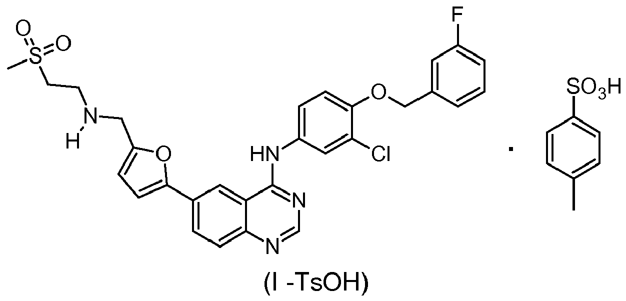
(B.) treatment of Lapatinib monotosylate respectively with caproic acid or caprylic acid.

9. Process for the preparation of the co-crystal of Lapatinib being Lapatinib monohydrochloride : adipic acid (1:1) of formula (Form III): 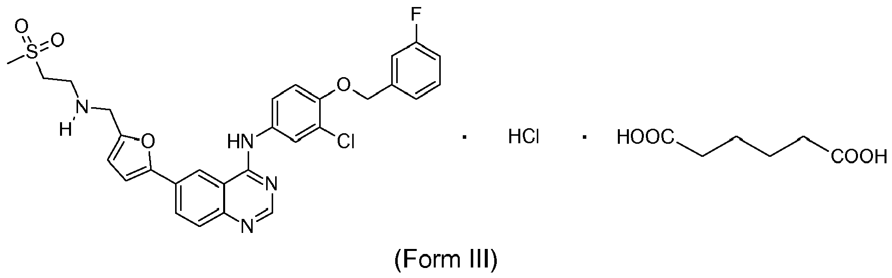 comprising the following steps:
(A.) reaction of Lapatinib of formula (I) with hydrochloric acid to provide Lapatinib monohydrochloride of formula (I - HCl): 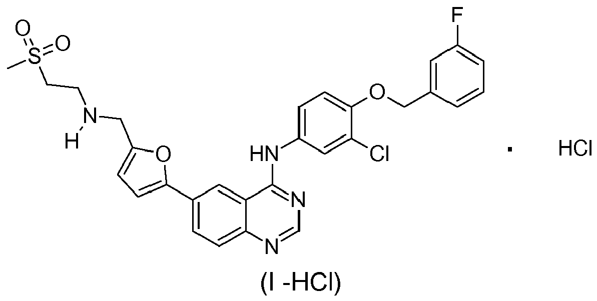 (B.) treatment of Lapatinib monohydrochloride with adipic acid.

10. Use of a salt of Lapatinib with a monoprotic acid in stoichiometry (1:1) for the preparation of co-crystals of Lapatinib.

11. Use according to the claim 10, wherein the of salt is Lapatinib monotosylate or Lapatinib monohydrochloride.

12. Use according anyone of the claims from 10 to 11 for the preparation of co-crystals of Lapatinib according to anyone of the claims from 1 to 7.

13. Co-crystal of Lapatinib according anyone of the claims from 1 to 7 for use in medicine.

14. Co-crystal of Lapatinib according anyone of the claims from 1 to 7 for use in a method for treatment of breast cancer and other solid tumours.

15. Pharmaceutical compositions comprising the co-crystal of Lapatinib according to anyone of the claims from 1 to 7 and one ore more excipients, carriers o diluents pharmaceutically acceptable.
